# FASCICULE DE BREVET EUROPEEN

(11) **EP 3 573 552 B1**
(45) Date de publication et mention de la délivrance du brevet: **09.12.2020**
(21) Numéro de dépôt: 18702816.2
(22) Date de dépôt: 23.01.2018
(51) Int. Cl.: A61B 17/70

(54) **SYSTEME D'OSTEOSYNTHESE RACHIDIENNE**
SYSTEM FÜR OSTEOSYNTHESE DER WIRBELSÄULE
SPINAL OSTEOSYNTHESIS SYSTEM

(30) Priorité: 25.01.2017 FR 1750615
(43) Date de publication de la demande: 04.12.2019
(73) Titulaire: Lape Medical, 74950 Scionzier (FR)
(72) Inventeur: GRADEL, Thomas, 74970 Marignier (FR)
(74) Mandataire: Cabinet Poncet
(86) Numéro de dépôt international: PCT/IB2018/050394
(87) Numéro de publication internationale: WO 2018/138627

(56) Documents cités:
- US-A1- 2005 113 831
- US-A1- 2009 062 860
- US-A1- 2012 150 185
- US-B2- 8 597 332

## Description

### DOMAINE TECHNIQUE DE L'INVENTION

La présente invention concerne le traitement du rachis, et concerne plus particulièrement un système d'ostéosynthèse rachidienne.

Un système d'ostéosynthèse rachidienne comprend généralement une tige vertébrale, un organe d'ancrage vertébral, un connecteur de liaison entre la tige vertébrale et l'organe d'ancrage vertébral, et des moyens de pression pour bloquer sélectivement ensemble la tige vertébrale, l'organe d'ancrage vertébral et le connecteur de liaison. De façon connue :
- le connecteur de liaison comprend un corps comportant des moyens de réception de l'organe d'ancrage vertébral orienté selon une première direction axiale,
- le connecteur de liaison comprend un logement de réception sensiblement cylindrique destiné à recevoir la tige vertébrale, se développant selon une deuxième direction axiale sensiblement perpendiculaire à la première direction axiale,
- le logement de réception comporte une ouverture latérale définissant deux bords et permettant l'introduction de la tige vertébrale dans le logement de réception transversalement ou radialement par rapport à la deuxième direction axiale, l'ouverture latérale ayant une largeur au moins égale au diamètre de la tige vertébrale.

Un exemple de système d'ostéosynthèse est par exemple décrit dans le document EP 1 011 504 B1. Dans ce document, le connecteur de liaison est monobloc et comprend un logement de réception sensiblement cylindrique capable de recevoir latéralement et retenir par encliquetage la tige vertébrale. Selon un premier mode opératoire, lors d'une opération, le chirurgien engage latéralement et encliquette une pluralité de connecteurs de liaison sur la tige vertébrale, puis rapporte les connecteurs de liaison sur des organes d'ancrage vertébral (en forme de vis).

L'encliquetage est procuré par l'élasticité du corps de connecteur de liaison, qui est monobloc. Il faut que cette élasticité soit suffisamment élevée pour autoriser un encliquetage sur la tige vertébrale sans trop d'efforts dans le logement de réception sensiblement cylindrique. Mais il faut également que cette élasticité soit suffisamment réduite pour retenir efficacement la tige vertébrale dans le logement de réception sensiblement cylindrique. Il en résulte que l'encliquetage est difficile à maîtriser.

En pratique, la tenue par encliquetage du connecteur de liaison sur la tige vertébrale s'avère toutefois très (trop) précaire, de sorte qu'il se produit souvent un détachement fortuit et intempestif du connecteur de liaison à l'écart de la tige vertébrale.

Il y a généralement au moins deux connecteurs de liaison sur une tige vertébrale. Lors du premier mode opératoire, le chirurgien installe une pluralité d'organes d'ancrage vertébral dans le rachis du patient, puis il pré-positionne les connecteurs de liaison sur la tige vertébrale avant de rapporter et fixer la tige vertébrale sur les organes d'ancrage vertébral au moyen des connecteurs de liaison et moyens de pression. L'écart relatif ainsi que l'orientation relative sur la tige vertébrale des connecteurs de liaison pré-positionnés doivent être ajustés à l'écart relatif et à l'orientation relative des organes d'ancrage vertébral lorsque le chirurgien rapporte la tige vertébrale et ses connecteurs de liaison sur la pluralité d'organes d'ancrage vertébral. Si la tenue par encliquetage du connecteur de liaison sur la tige vertébrale s'avère parfois satisfaisante, il est alors difficile de décaler un connecteur de liaison par glissement le long de la tige vertébrale ou de faire tourner le connecteur de liaison sur la tige vertébrale sans provoquer un détachement intempestif du connecteur de liaison pré-positionné sur la tige vertébrale.

En outre, le connecteur de liaison et l'organe d'ancrage vertébral du document EP 1 011 504 B1 sont conformés de telle sorte que, avant blocage du connecteur sur la tige et lorsque le connecteur de liaison est engagé sur l'organe d'ancrage vertébral, la tige vertébrale ne peut pas s'échapper latéralement hors du connecteur. Il en résulte que le chirurgien est obligé d'engager la tige vertébrale dans le connecteur de liaison avant d'engager ce dernier sur l'organe d'ancrage vertébral (selon le premier mode opératoire explicité précédemment). Or, de nombreux chirurgiens préfèrent, selon un deuxième mode opératoire, commencer par engager les connecteurs de liaison sur les organes d'ancrage vertébral avant d'engager la tige vertébrale dans les connecteurs de liaison. Cela évite en effet d'avoir à positionner approximativement les connecteurs de liaison sur la tige vertébrale et de risquer une perte par détachement intempestif lors de l'ajustement des connecteurs de liaison le long et autour la tige vertébrale en fonction des positions relatives des organes d'ancrage vertébral.

Les documents US 8,597,332 B2 et US 2009/0062860 A1 décrivent d'autres systèmes d'ostéosynthèse. Le document US 2009/0062860 A1 décrit plus particulièrement un système d'ostéosynthèse selon le préambule de la revendication 1. Dans ces documents, une rétention précaire de la tige vertébrale dans le logement de réception est possible, afin de permettre de décaler un connecteur de liaison par glissement le long de la tige vertébrale ou de faire tourner le connecteur de liaison sur la tige vertébrale, avant une immobilisation en mouvement complète par verrouillage de la tige vertébrale dans le logement de réception. Mais cette rétention précaire résulte alors d'un encliquetage procuré par l'élasticité du corps de connecteur de liaison lui-même, ce qui mène aux mêmes inconvénients que ceux décrits en lien avec le document EP 1 011 504 B1.

### EXPOSE DE L'INVENTION

Un problème proposé par la présente invention est de fournir un système d'ostéosynthèse rachidienne dans lequel le connecteur autorise le chirurgien à mettre indifféremment en œuvre les premier et deuxième modes opératoires décrits précédemment.

Un autre problème proposé par l'invention est d'assurer un encliquetage maîtrisé, afin de limiter les risques d'échappement intempestif des connecteurs de liaison et les risques de perte afférente, tout en évitant au chirurgien d'avoir à exercer des efforts trop pénibles pour rapporter les connecteurs de liaison sur la tige vertébrale.

Pour atteindre ces objets ainsi que d'autres, l'invention propose un système d'ostéosynthèse rachidienne comprenant une tige vertébrale, un organe d'ancrage vertébral, un connecteur de liaison entre la tige vertébrale et l'organe d'ancrage vertébral, et des moyens de pression pour bloquer sélectivement ensemble la tige vertébrale, l'organe d'ancrage vertébral et le connecteur de liaison, dans lequel :
- le connecteur de liaison comprend un corps comportant des moyens de réception de l'organe d'ancrage vertébral orienté selon une première direction axiale,
- le corps comprend un logement de réception sensiblement cylindrique destiné à recevoir la tige vertébrale, se développant selon une deuxième direction axiale sensiblement perpendiculaire à la première direction axiale,
- le logement de réception comporte une ouverture latérale définissant deux bords et permettant l'introduction de la tige vertébrale dans le logement de réception transversalement ou radialement par rapport à la deuxième direction axiale, l'ouverture latérale ayant une largeur au moins égale au diamètre de la tige vertébrale,
- le connecteur de liaison comporte un organe de verrouillage déplaçable par rapport au corps du connecteur de liaison, l'organe de verrouillage ayant une position de libération dans laquelle l'organe de verrouillage autorise le passage de la tige vertébrale à travers l'ouverture latérale du logement de réception,
- les moyens de pression permettent de s'opposer sélectivement à un déplacement de l'organe de verrouillage vers sa position de libération ;
selon l'invention :
- l'organe de verrouillage est déplaçable par rapport au corps du connecteur de liaison depuis sa position de libération vers une position de rétention, dans laquelle l'organe de verrouillage s'oppose élastiquement au passage de la tige vertébrale à travers l'ouverture latérale du logement de réception par des moyens de rappel élastique qui pressent l'organe de verrouillage en appui contre la tige vertébrale contenue dans le logement de réception selon un premier effort de serrage prédéterminé,
- les moyens de pression permettent de solliciter sélectivement l'organe de verrouillage vers une position de verrouillage dans laquelle l'organe de verrouillage est pressé en appui contre la tige vertébrale contenue dans le logement de réception selon un deuxième effort de serrage supérieur au premier effort de serrage prédéterminé exercé par les moyens de rappel élastique.

Un tel système d'ostéosynthèse autorise le deuxième mode opératoire décrit précédemment.

L'organe de verrouillage est distinct du corps du connecteur de liaison, de sorte que la force de rappel élastique de l'organe de verrouillage vers sa position de rétention peut être facilement dimensionnée pour ajuster le premier effort de serrage prédéterminé. Ce premier effort de serrage prédéterminé obligera le chirurgien à exercer sur le connecteur de liaison une certaine force (pas trop élevée) pour encliqueter le connecteur de liaison sur la tige vertébrale et déplacer le connecteur de liaison à coulissement et/ou en rotation (liaison de type pivot glissant) sur la tige vertébrale. Cette liaison de type pivot glissant permet une orientation et un coulissement aisés du connecteur de liaison sur la tige vertébrale, tout en assurant un freinage relatif suffisant entre le connecteur de liaison et la tige vertébrale, et en limitant efficacement les risques de séparation intempestive entre le connecteur de liaison et la tige vertébrale, de sorte que le premier mode opératoire peut également être mis en oeuvre. En même temps, ce premier effort de serrage prédéterminé procure une rétention satisfaisante du connecteur de liaison sur la tige vertébrale pour éviter un échappement intempestif de cette dernière hors du logement de réception. L'organe de verrouillage est également distinct des moyens de pression.

Lorsque le chirurgien souhaite bloquer ensemble la tige vertébrale, l'organe d'ancrage vertébral et le connecteur de liaison, il manœuvre les moyens de pression afin que ces derniers sollicitent l'organe de verrouillage en pression contre la tige vertébrale contenue dans le logement de réception selon un deuxième effort de serrage supérieur au premier effort de serrage prédéterminé. Les moyens de pression s'opposent alors à un déplacement de l'organe de verrouillage vers sa position de libération, et bloquent ensemble la tige vertébrale, l'organe d'ancrage vertébral et le connecteur de liaison de façon fiable.

Avantageusement, on peut prévoir que :
- l'organe de verrouillage comprend un mors,
- en position de rétention et en position de verrouillage, le mors et un bord opposé du logement de réception sont distants l'un de l'autre d'une distance inférieure au diamètre de la tige vertébrale,
- en position de libération, le mors et ledit bord opposé du logement de réception sont distants l'un de l'autre d'une distance supérieure au diamètre de la tige vertébrale.

L'organe de verrouillage a ainsi une structure simple et sa mise en œuvre est très intuitive pour le chirurgien.

Avantageusement, l'organe de verrouillage peut être disposé à pivotement autour d'un axe de pivot orienté selon une troisième direction axiale parallèle à la deuxième direction axiale. On obtient ainsi de façon simple et efficace le mouvement relatif entre le mors et le corps du connecteur de liaison.

De préférence, l'organe de verrouillage peut comporter un premier bras s'étendant radialement à l'écart de l'axe de pivot et muni d'une facette d'immobilisation, de préférence au voisinage de son extrémité libre, contre laquelle viennent porter en appui les moyens de pression pour solliciter l'organe de verrouillage vers sa position de verrouillage. La distance entre la facette d'immobilisation et l'axe de pivot permet un effet de levier pour immobiliser efficacement l'organe de verrouillage (et donc son mors) en position de rétention.

Avantageusement, on peut prévoir que :
- l'organe de verrouillage comporte un deuxième bras s'étendant radialement à l'écart de l'axe de pivot depuis une extrémité fixe et se terminant par une extrémité libre,
- entre son extrémité fixe et son extrémité libre, le deuxième bras est pourvu d'un tronçon plus facilement élastiquement déformable que le premier bras, et qui n'est pas au contact du corps du connecteur de liaison,
- le deuxième bras est en appui au voisinage de son l'extrémité libre sur le corps du connecteur de liaison, et est conformé pour rappeler élastiquement l'organe de verrouillage vers sa position de rétention.

De la sorte, on dispose de moyens de rappel élastique qui font partie intégrante de l'organe de verrouillage, pour une bonne compacité du système. Et on limite ainsi le nombre de pièces à fabriquer et à assembler.

De préférence, pour augmenter la retenue de la tige vertébrale dans le logement de réception, ce dernier peut être pourvu intérieurement de reliefs de rétention.

De préférence, les moyens de réception de l'organe d'ancrage vertébral peuvent comprendre un trou traversant le corps et orienté selon la première direction axiale. De tels moyens de réception ont une structure simple et robuste. Avantageusement, on peut prévoir que :
- l'organe d'ancrage vertébral comprend un premier tronçon fileté extérieurement destiné à pénétrer dans une vertèbre, se terminant par un épaulement à partir duquel s'étend un deuxième tronçon destiné à recevoir les moyens de pression,
- l'épaulement est destiné à recevoir en appui le connecteur de liaison en étant pressé par les moyens de pression.

De préférence, on peut prévoir que :
- le deuxième tronçon de l'organe d'ancrage vertébral est fileté extérieurement,
- les moyens de pression comprennent un écrou destiné à être vissé sur le deuxième tronçon de l'organe d'ancrage vertébral.

### DESCRIPTION SOMMAIRE DES DESSINS

D'autres objets, caractéristiques et avantages de la présente invention ressortiront de la description suivante de modes de réalisation particuliers, faite en relation avec les figures jointes, parmi lesquelles :
- la figure 1 est une vue en perspective d'un mode de réalisation particulier de système d'ostéosynthèse selon l'invention, avec la tige vertébrale située hors du logement de réception du connecteur de liaison ;
- la figure 2 est une vue de face du système d'ostéosynthèse de la figure 1 ;
- la figure 3 est une vue partielle de face et en coupe du système d'ostéosynthèse de la figure 1 ;
- la figure 4 est une vue de face du système d'ostéosynthèse de la figure 1, avec la tige vertébrale en cours d'introduction dans le logement de réception du connecteur de liaison ;
- la figure 5 est une vue partielle de face et en coupe du système d'ostéosynthèse de la figure 4 ;
- la figure 6 est une vue de face du système d'ostéosynthèse de la figure 1, avec la tige vertébrale située dans le logement de réception du connecteur de liaison ;
- la figure 7 est une vue partielle de face et en coupe du système d'ostéosynthèse de la figure 6 ;
- la figure 8 est une vue de face du système d'ostéosynthèse de la figure 1, avec les moyens de pression pressant l'organe de verrouillage contre la tige vertébrale contenue dans le logement de réception du connecteur de liaison ;
- la figure 9 est une vue partielle de face et en coupe du système d'ostéosynthèse de la figure 8.

### DESCRIPTION DES MODES DE REALISATION PREFERES

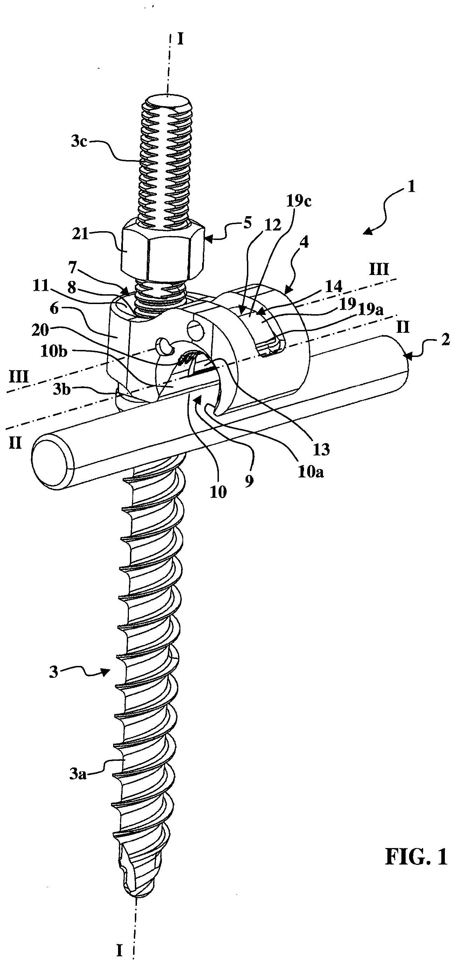

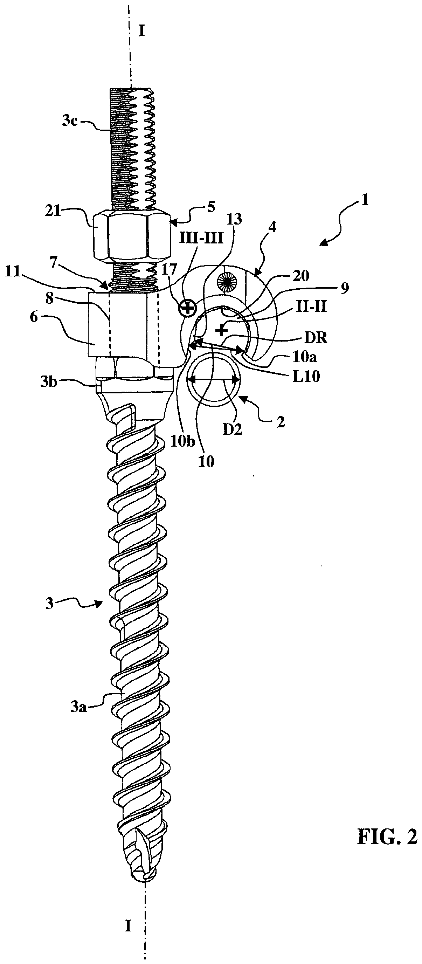

Sur les figures 1 à 9 est illustré un mode de réalisation particulier de système d'ostéosynthèse rachidienne 1 selon l'invention. Celui-ci comprend une tige vertébrale 2, un organe d'ancrage vertébral 3, un connecteur de liaison 4 entre la tige vertébrale 2 et l'organe d'ancrage vertébral 3, et des moyens de pression 5 pour bloquer ensemble la tige vertébrale 2, l'organe d'ancrage vertébral 3 et le connecteur de liaison 4.

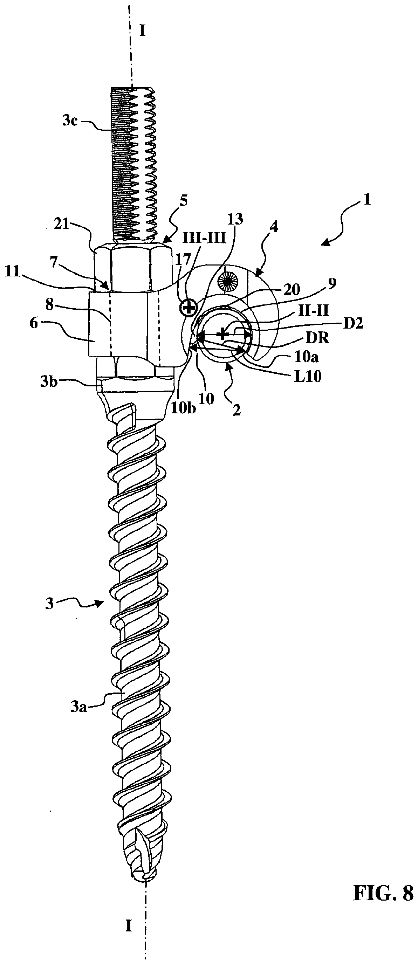

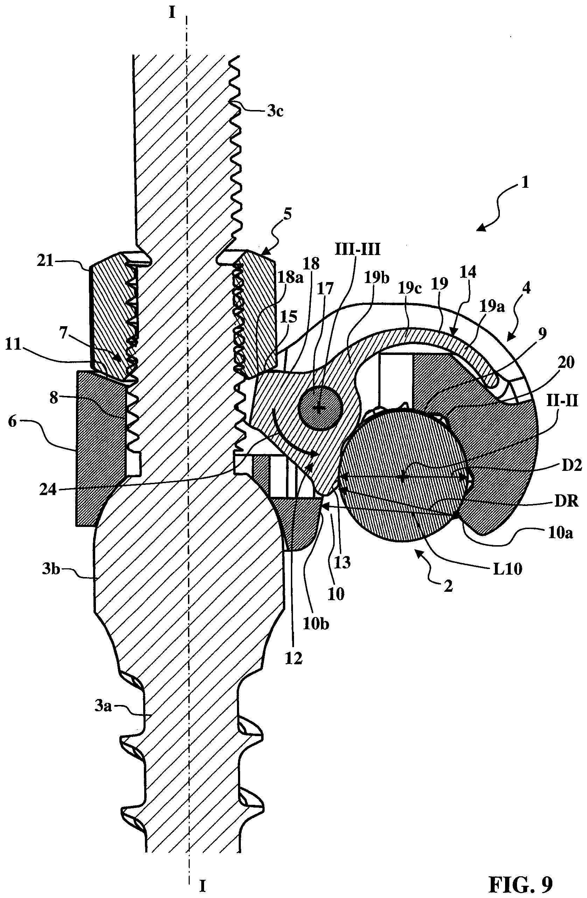

L'organe d'ancrage vertébral 3 comprend un premier tronçon fileté 3a destiné à pénétrer dans une vertèbre, se terminant par un épaulement 3b à partir duquel s'étend un deuxième tronçon 3c fileté extérieurement destiné à recevoir les moyens de pression 5. L'épaulement 3b est destiné à recevoir en appui le connecteur de liaison 4 en étant pressé par les moyens de pression 5 (figures 8 et 9).

Les moyens de pression 5 comprennent un écrou 21 destiné à être vissé sur le deuxième tronçon 3c fileté de l'organe d'ancrage vertébral 3.

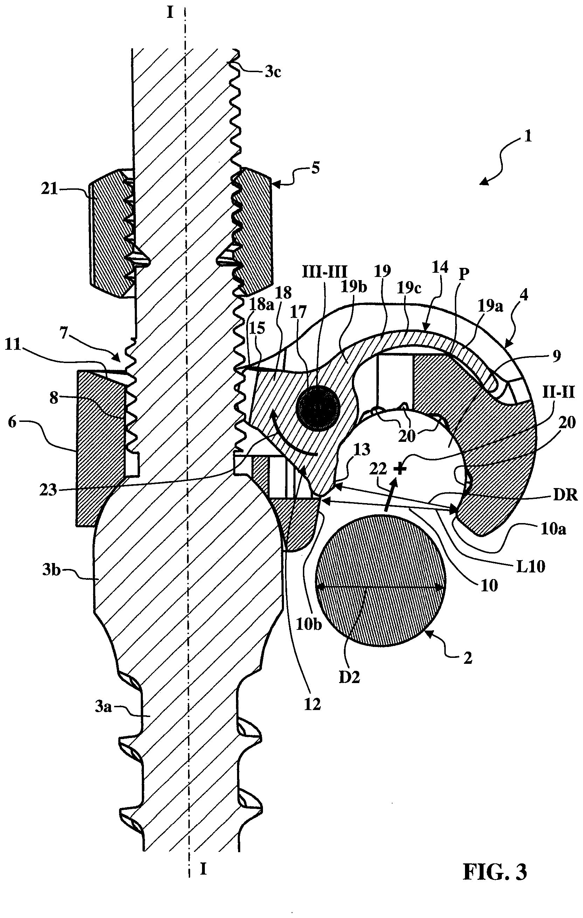

Sur les figures 1 à 3, on voit que le connecteur de liaison 4 comprend un corps 6 comportant des moyens de réception 7 de l'organe d'ancrage vertébral 3 orienté selon une première direction axiale I-I. En l'espèce, les moyens de réception 7 comprennent un comprennent un trou 8 traversant le corps 6 et orienté selon la première direction axiale I-I (plus particulièrement visible sur la figure 3).

Le corps 6 comprend un logement de réception 9 sensiblement cylindrique destiné à recevoir la tige vertébrale 2. Le logement de réception 9 se développe selon une deuxième direction axiale II-II sensiblement perpendiculaire à la première direction axiale I-I.

Le logement de réception 9 comporte une ouverture latérale 10 définissant deux bords 10a et 10b, et permettant l'introduction de la tige vertébrale 2 dans le logement de réception 9 transversalement ou radialement par rapport à la deuxième direction axiale II-II. L'ouverture latérale 10 a une largeur L10 au moins égale au diamètre D2 de la tige vertébrale 2.

Le connecteur de liaison 4 comporte une surface d'appui 11 contre laquelle peuvent venir en appui les moyens de pression 5.

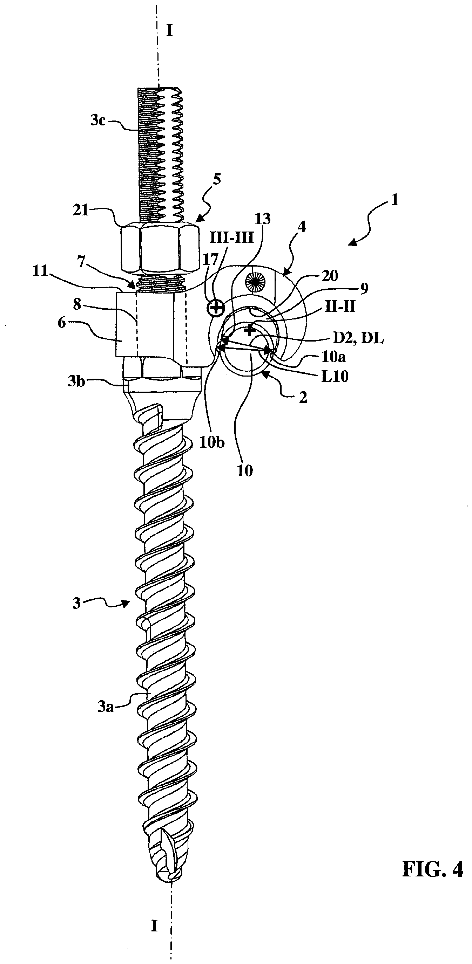

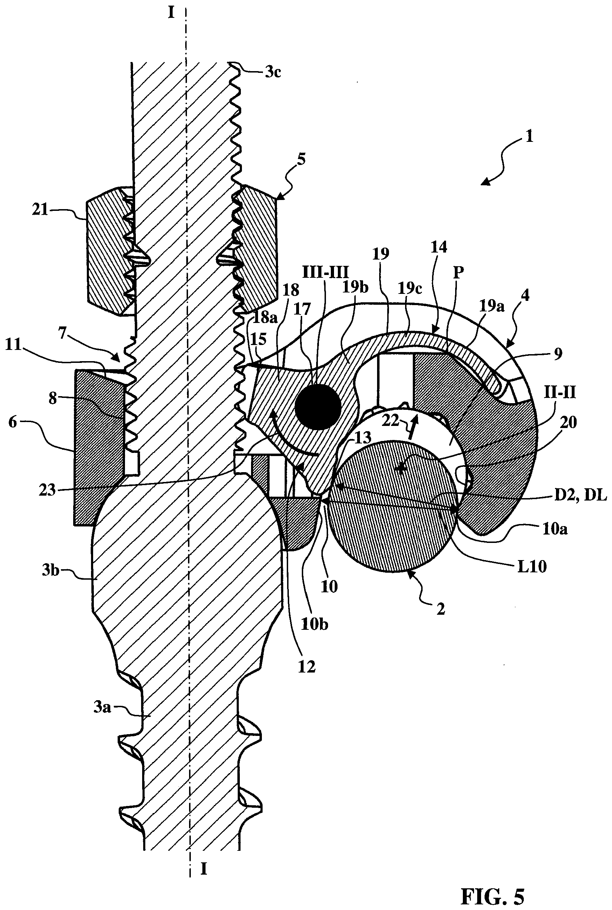

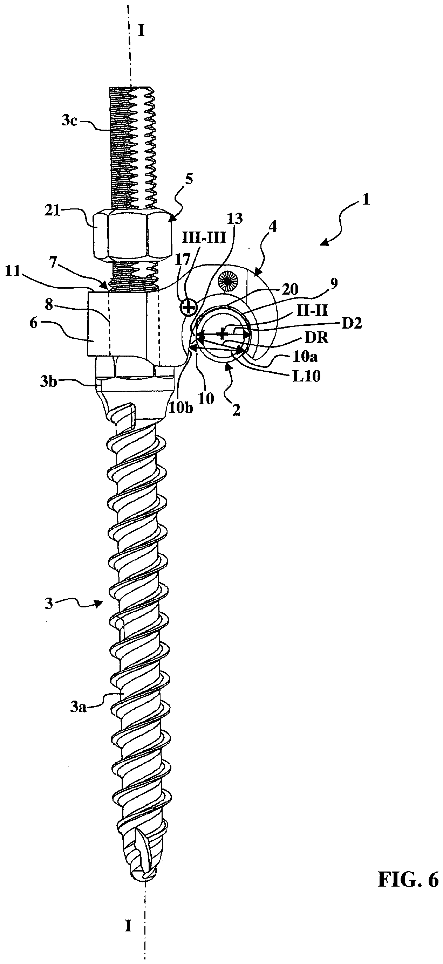

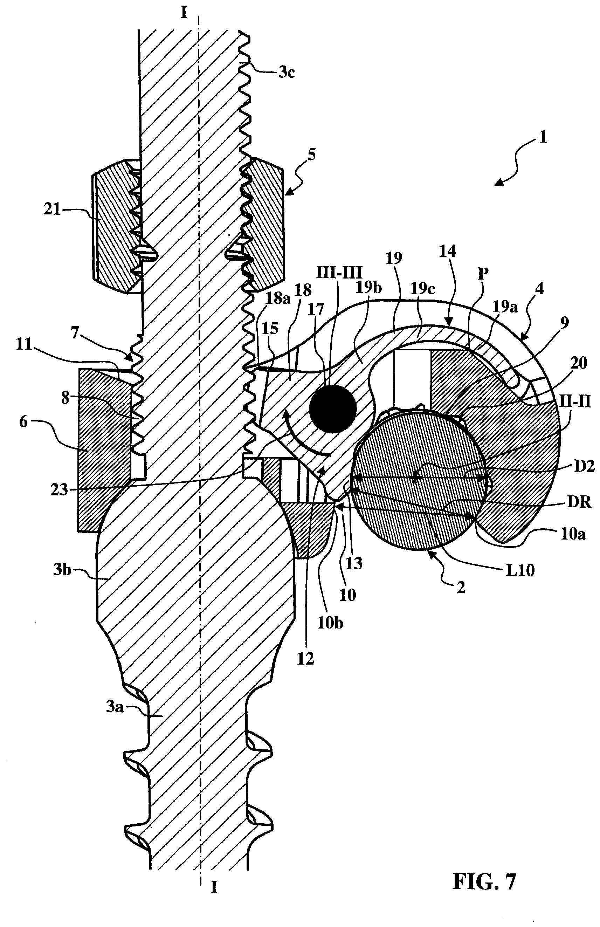

Comme on le voit plus particulièrement sur la figure 3, le connecteur de liaison 4 comporte un organe de verrouillage 12 déplaçable par rapport au corps 6 du connecteur de liaison 4 entre une position de rétention (figures 6 et 7), dans laquelle l'organe de verrouillage 12 s'oppose au passage de la tige vertébrale 2 dans l'ouverture radiale 10 du logement de réception 9, et une position de libération (figures 4 et 5), dans laquelle l'organe de verrouillage 12 autorise le passage de la tige vertébrale 2 dans l'ouverture latérale 10 du logement de réception 9.

En position de rétention (figures 6 et 7), des moyens de rappel élastique 14 pressent l'organe de verrouillage 12 en appui contre la tige vertébrale 2 contenue dans le logement de réception 9 selon un premier effort de serrage prédéterminé.

Comme on le voit plus particulièrement sur les figures 8 et 9, les moyens de pression 5 permettent de s'opposer à un déplacement de l'organe de verrouillage 12 vers sa position de libération (figures 4 et 5), et permettent de solliciter l'organe de verrouillage 12 vers une position de verrouillage (figures 8 et 9) dans laquelle l'organe de verrouillage 12 est pressé contre la tige vertébrale 2 contenue dans le logement de réception 9 selon un deuxième effort de serrage supérieur au premier effort de serrage prédéterminé.

L'organe de verrouillage 12 comprend un mors 13. En position de rétention (figures 6 et 7) et en position de verrouillage (figures 8 et 9), le mors 13 et le bord opposé 10a du logement de réception 10 sont distants l'un de l'autre d'une distance DR inférieure au diamètre D2 de la tige vertébrale 2. En position de libération (figures 4 et 5), le mors 13 et ledit bord opposé 10a du logement de réception 9 sont distants l'un de l'autre d'une distance DL supérieure ou égale au diamètre D2 de la tige vertébrale 2 (sur les figures 4 et 5, la distance DL est égale au diamètre D2).

L'organe de verrouillage 12 comporte une facette d'immobilisation 15, destinée à recevoir en appui les moyens de pression 5 lorsque les moyens de pression 5 sont déplacés par vissage vers le connecteur de liaison 4. Lorsque les moyens de pression 5 sont en appui sur la facette d'immobilisation 15, ils s'opposent à un déplacement du mors 13 vers sa position de libération (figures 4 et 5). Les moyens de pression permettent également de solliciter l'organe de verrouillage 12 vers sa position de verrouillage (figures 8 et 9) dans laquelle l'organe de verrouillage 12 est pressé contre la tige vertébrale 2 contenue dans le logement de réception 9 selon un deuxième effort de serrage supérieur au premier effort de serrage prédéterminé.

L'organe de verrouillage 12 est disposé à pivotement autour d'un axe de pivot 17 orienté selon une troisième direction axiale III-III parallèle à la deuxième direction axiale II-II.

Comme illustré de façon plus détaillée sur les vues en coupe des figures 3, 5, 7 et 9, l'organe de verrouillage 12 comporte un premier bras 18 s'étendant radialement à l'écart de l'axe de pivot 17 et muni de la facette d'immobilisation 15, de préférence au voisinage de son extrémité libre 18a. Les moyens de pression 5 viennent porter en appui contre la facette d'immobilisation 15 pour solliciter l'organe de verrouillage 12 vers sa position de verrouillage (figure 8 et 9).

On voit également que les moyens de rappel élastique 14 comportent un deuxième bras 19 s'étendant radialement depuis une extrémité fixe 19b à l'écart de l'axe de pivot 17 et se terminant par une extrémité libre 19a. Pour rappeler élastiquement l'organe de verrouillage 12 vers sa position de rétention, le deuxième bras 19 est en appui au voisinage de son extrémité libre 19a sur le corps 6 du connecteur de liaison 4 (point d'appui P). Entre son extrémité fixe 19b et son extrémité libre 19a, le deuxième bras 19 est pourvu d'un tronçon 19c intermédiaire plus facilement élastiquement déformable que le premier bras 18. Ce tronçon 19c intermédiaire n'est pas au contact du corps 6 du connecteur de liaison 4, pour pouvoir fléchir selon une certaine amplitude.

Le logement de réception 9 est pourvu intérieurement de reliefs de rétention 20.

L'utilisation du système d'ostéosynthèse rachidienne 1 selon le deuxième mode opératoire va désormais être explicitée à l'aide des figures 3, 5, 7 et 9.

Le chirurgien a préalablement inséré par vissage l'organe d'ancrage vertébral 3 dans la vertèbre d'un patient. Il a ensuite engagé le connecteur de liaison 4 en faisant pénétrer le deuxième tronçon fileté 3c dans le trou 8 des moyens de réception 7, jusqu'à amener le connecteur de liaison 4 en butée contre l'épaulement 3b. Le chirurgien a également engagé par vissage l'écrou 21 des moyens de pression 5 sur le deuxième tronçon fileté 3c, mais sans toutefois venir jusqu'au contact du connecteur de liaison 4. On se trouve alors dans la configuration illustrée sur la figure 3, avec le mors 13 de l'organe de verrouillage 12 disposé en position de rétention.

Le chirurgien approche alors la tige vertébrale 2 de l'ouverture latérale 10 pour l'insérer dans le logement de réception 9 selon un mouvement transversal illustré par la flèche 22 (figure 3).

Lors de ce mouvement, la distance DR entre le mors 13 et le bord opposé 10a étant inférieure au diamètre D2 de la tige vertébrale 2, la tige vertébrale 2 vient simultanément au contact du mors 13 et du bord opposé 10a.

Le chirurgien applique alors un effort pour poursuivre le mouvement illustré par la flèche 22. Cet effort a pour effet de faire pivoter l'organe de verrouillage 12 autour de la troisième direction axiale III-III selon le mouvement illustré par la flèche 23 et l'amener en position de libération telle qu'illustrée sur la figure 5, en déformant élastiquement le tronçon 19c.

Sur la figure 5, la distance DL entre le mors 13 et le bord opposé 10a étant égale au diamètre D2 de la tige vertébrale 2, la tige vertébrale 2 pénètre dans le logement de réception 9 lorsque le chirurgien poursuit le mouvement transversal illustré par la flèche 22.

On parvient alors à la configuration illustrée sur la figure 7, dans laquelle la tige vertébrale 2 a été reçue dans le logement de réception 9. Après cette réception, le tronçon 19c des moyens de rappel élastique 14 a automatiquement rappelé l'organe de verrouillage 12 et son mors 13 en position de rétention. La tige vertébrale 2 est ainsi retenue captive dans le logement de réception 9, avec le mors 13 en appui contre la tige vertébrale 2 selon le premier effort de serrage prédéterminé.

Ce premier effort de serrage prédéterminé est suffisamment fort pour éviter une extraction intempestive de la tige vertébrale 2 hors du logement de réception 9, et est suffisamment modéré pour autoriser :
- un coulissement et/ou une rotation du connecteur de liaison sur la tige vertébrale 2,
- une extraction volontaire de la tige vertébrale 2 hors du logement de réception 9 par un mouvement inverse de celui illustré par la flèche 22, qui aura pour effet de déformer à nouveau élastiquement le tronçon 19c pour laisser pivoter l'organe de verrouillage 12 (selon un mouvement inverse de celui illustré par la flèche 23) et amener le mors 13 en position de libération (figure 5).

Une fois que le chirurgien a fini la mise en place de la tige vertébrale 2, il visse l'écrou 21 jusqu'à l'amener en appui contre la facette d'immobilisation 15 pour presser encore plus fortement le mors 13 contre la tige vertébrale 2 (figures 8 et 9). Dans le mode de réalisation illustré sur les figures 1 à 9, le serrage de l'écrou 21 contre la facette d'immobilisation 15 fait légèrement pivoter l'organe de verrouillage 12 autour de son axe de pivot 17 dans le sens illustré par la flèche 24 sur la figure 9 pour amener l'organe de verrouillage 12 dans la position de verrouillage des figures 8 et 9. Sur ces figures, on constate que le déplacement de l'organe de verrouillage 12 par l'écrou 21 a même très légèrement écarté l'extrémité libre 19a du deuxième bras 19 à l'écart du corps 6, de sorte que le deuxième bras 19 n'est plus en appui sur le corps 6.

Le fonctionnement du connecteur de liaison 4 dans le cadre du premier mode opératoire est similaire.

Lors de ce premier mode opératoire, le chirurgien pré-positionne le connecteur de liaison 4 en encliquetant ce dernier sur la tige vertébrale 2. Après encliquetage, le connecteur est dans une configuration similaire à celle des figures 6 et 7 avec l'organe de verrouillage 12 en position de rétention.

La tige vertébrale 2 est ainsi retenue captive dans le logement de réception 9 avec le mors 13 en appui élastique contre la tige vertébrale 2 selon le premier effort de serrage prédéterminé.

Ce premier effort de serrage prédéterminé est suffisamment fort pour éviter une extraction intempestive de la tige vertébrale 2 hors du logement de réception 9, et est suffisamment modéré pour autoriser :
- un coulissement et/ou une rotation du connecteur de liaison 4 sur la tige vertébrale 2, afin d'ajuster la position et l'orientation du connecteur de liaison 4 le long et autour la tige vertébrale 2 en fonction de la position et l'orientation de l'organe d'ancrage vertébral 3,
- une extraction volontaire de la tige vertébrale 2 hors du logement de réception 9.

Après avoir pré-positionné sur la tige vertébrale 2 les connecteurs de liaison 4, le chirurgien rapporte le sous-ensemble d'un seul tenant constitué par la tige vertébrale 2 et les connecteurs de liaison 4 sur les organes d'ancrage vertébral 3. Pour ce faire, le chirurgien ajuste l'écart relatif ainsi que l'orientation relative sur la tige vertébrale 2 des connecteurs de liaison 4 pré-positionnés pour les mettre en adéquation avec l'écart relatif et l'orientation relative des organes d'ancrage vertébral 3.

Lors de cet ajustement, il est aisé de décaler un connecteur de liaison 4 par glissement le long de la tige vertébrale 2 ou de faire tourner le connecteur de liaison 4 sur la tige vertébrale 2 sans provoquer un détachement intempestif du connecteur de liaison 4 pré-positionné sur la tige vertébrale 2, car l'organe de verrouillage 4 n'est en appui contre la tige vertébrale 2 que selon le premier effort de serrage prédéterminé.

Une fois que le chirurgien a fini la mise en place du sous-ensemble d'un seul tenant constitué par la tige vertébrale 2 et les connecteurs de liaison 4 sur les organes d'ancrage vertébral 3 (par engagement du deuxième tronçon 3c dans le trou 8 des connecteurs de liaison 4), il visse des écrous 21 sur les deuxièmes tronçons 3c jusqu'à amener les écrous 21 en appui contre la facette d'immobilisation 15 pour déplacer les organes de verrouillage 12 en position de verrouillage (figures 8 et 9). Chaque mors 13 presse ainsi encore plus fortement (selon un deuxième effort de serrage) la tige vertébrale 2 et immobilise définitivement la tige vertébrale 2 par rapport aux organes d'ancrage vertébral 3.

La présente invention n'est pas limitée aux modes de réalisation qui ont été explicitement décrits, mais elle en inclut les diverses variantes et généralisations contenues dans le domaine des revendications ci-après.

## Revendications

1. Système d'ostéosynthèse rachidienne (1) comprenant une tige vertébrale (2), un organe d'ancrage vertébral (3), un connecteur de liaison (4) entre la tige vertébrale (2) et l'organe d'ancrage vertébral (3), et des moyens de pression (5) pour bloquer sélectivement ensemble la tige vertébrale (2), l'organe d'ancrage vertébral (3) et le connecteur de liaison (4), dans lequel :
- le connecteur de liaison (4) comprend un corps (6) comportant des moyens de réception (7) de l'organe d'ancrage vertébral (3) orienté selon une première direction axiale (1-1),
- le corps (6) comprend un logement de réception (9) sensiblement cylindrique destiné à recevoir la tige vertébrale (2), se développant selon une deuxième direction axiale (II-II) sensiblement perpendiculaire à la première direction axiale (I-I),
- le logement de réception (9) comporte une ouverture latérale (10) définissant deux bords (10a, 10b) et permettant l'introduction de la tige vertébrale (2) dans le logement de réception (9) transversalement ou radialement par rapport à la deuxième direction axiale (II-II), l'ouverture latérale (10) ayant une largeur (L10) au moins égale au diamètre (D2) de la tige vertébrale (2),
- le connecteur de liaison (4) comporte un organe de verrouillage (12) déplaçable par rapport au corps (6) du connecteur de liaison (4), l'organe de verrouillage (12) ayant une position de libération dans laquelle l'organe de verrouillage (12) autorise le passage de la tige vertébrale (2) à travers l'ouverture latérale (10) du logement de réception (9),
- les moyens de pression (5) permettent de s'opposer sélectivement à un déplacement de l'organe de verrouillage (12) vers sa position de libération,
**caractérisé en ce que** :
- l'organe de verrouillage (12) est déplaçable par rapport au corps (6) du connecteur de liaison (4) depuis sa position de libération vers une position de rétention, dans laquelle l'organe de verrouillage (12) s'oppose élastiquement au passage de la tige vertébrale (2) à travers l'ouverture latérale (10) du logement de réception (9) par des moyens de rappel élastique (14) qui pressent l'organe de verrouillage (12) en appui contre la tige vertébrale (2) contenue dans le logement de réception (9) selon un premier effort de serrage prédéterminé,
- les moyens de pression (5) permettent de solliciter sélectivement l'organe de verrouillage (12) vers une position de verrouillage dans laquelle l'organe de verrouillage (12) est pressé en appui contre la tige vertébrale (2) contenue dans le logement de réception (9) selon un deuxième effort de serrage supérieur au premier effort de serrage prédéterminé exercé par les moyens de rappel élastique (14).

2. Système d'ostéosynthèse rachidienne (1) selon la revendication 1, **caractérisé en ce que** :
- l'organe de verrouillage (12) comprend un mors (13),
- en position de rétention et en position de verrouillage, le mors (13) et un bord (10a) opposé du logement de réception (9) sont distants l'un de l'autre d'une distance (DR) inférieure au diamètre (D2) de la tige vertébrale (2),
- en position de libération, le mors (13) et ledit bord (10a) opposé du logement de réception (9) sont distants l'un de l'autre d'une distance (DL) supérieure au diamètre (D2) de la tige vertébrale (2).

3. Système d'ostéosynthèse rachidienne (1) selon l'une des revendications 1 ou 2, **caractérisé en ce que** l'organe de verrouillage (12) est disposé à pivotement autour d'un axe de pivot (17) orienté selon une troisième direction axiale (III-III) parallèle à la deuxième direction axiale (II-II).

4. Système d'ostéosynthèse rachidienne (1) selon la revendication 3, **caractérisé en ce que** l'organe de verrouillage (12) comporte un premier bras (18) s'étendant radialement à l'écart de l'axe de pivot (17) et muni d'une facette d'immobilisation (15), de préférence au voisinage de son extrémité libre (18a), contre laquelle viennent porter en appui les moyens de pression (5) pour solliciter l'organe de verrouillage (12) vers sa position de verrouillage.

5. Système d'ostéosynthèse rachidienne (1) selon la revendication 4, **caractérisé en ce que** :
- l'organe de verrouillage (12) comporte un deuxième bras (19) s'étendant radialement à l'écart de l'axe de pivot (17) depuis une extrémité fixe (19b) et se terminant par une extrémité libre (19a),
- entre son extrémité fixe (19b) et son extrémité libre (19a), le deuxième bras (19) est pourvu d'un tronçon (19c) plus facilement élastiquement déformable que le premier bras (18), et qui n'est pas au contact du corps (6) du connecteur de liaison (4),
- le deuxième bras (19) est en appui au voisinage de son l'extrémité libre (19a) sur le corps (6) du connecteur de liaison (4), et est conformé pour rappeler élastiquement l'organe de verrouillage (12) vers sa position de rétention.

6. Système d'ostéosynthèse rachidienne (1) selon l'une quelconque des revendications 1 à 5, **caractérisé en ce que** le logement de réception (9) est pourvu intérieurement de reliefs de rétention (20).

7. Système d'ostéosynthèse rachidienne (1) selon l'une quelconque des revendications 1 à 6, **caractérisé en ce que** les moyens de réception (7) de l'organe d'ancrage vertébral (3) comprennent un trou (8) traversant le corps (6) et orienté selon la première direction axiale (I-I).

8. Système d'ostéosynthèse rachidienne (1) selon l'une quelconque des revendications 1 à 7, **caractérisé en ce que** :
- l'organe d'ancrage vertébral (3) comprend un premier tronçon (3a) fileté extérieurement destiné à pénétrer dans une vertèbre, se terminant par un épaulement (3b) à partir duquel s'étend un deuxième tronçon (3c) destiné à recevoir les moyens de pression (5),
- l'épaulement (3b) est destiné à recevoir en appui le connecteur de liaison (4) en étant pressé par les moyens de pression (5).

9. Système d'ostéosynthèse rachidienne (1) selon la revendication 8, **caractérisé en ce que** :
- le deuxième tronçon (3c) de l'organe d'ancrage vertébral (3) est fileté extérieurement,
- les moyens de pression (5) comprennent un écrou (21) destiné à être vissé sur le deuxième tronçon (3c) de l'organe d'ancrage vertébral (3).

## Patentansprüche

1. System für Osteosynthese der Wirbelsäule (1), umfassend einen Wirbelstab (2), ein Wirbelverankerungsorgan (3), einen Anschlussverbinder (4) zwischen dem Wirbelstab (2) und dem Wirbelverankerungsorgan (3) und Mittel zur Druckausübung (5) zum selektiven Blockieren des Wirbelstabs (2), des Wirbelverankerungsorgans (3) und des Anschlussverbinders (4), wobei:
- der Anschlussverbinder (4) einen Körper (6) umfasst, der Mittel zum Empfangen (7) des gemäß einer ersten axialen Richtung (I-I) orientierten Wirbelverankerungsorgans (3) aufweist,
- der Körper (6) ein im Wesentlichen zylindrisches Lager zum Empfangen (9) umfasst, das zur Aufnahme des Wirbelstabs (2) vorgesehen ist und sich gemäß einer zweiten axialen Richtung (II-II) im Wesentlichen senkrecht zur ersten axialen Richtung (I-I) entwickelt,
- das Lager zum Empfangen (9) eine seitliche Öffnung (10) aufweist, die zwei Kanten (10a, 10b) definiert und das Einführen des Wirbelstabs (2) in das Lager zum Empfangen (9) quer oder radial relativ zur zweiten axialen Richtung (II-II) erlaubt, wobei die seitliche Öffnung (10) eine Breite (L10) mindestens entsprechend dem Durchmesser (D2) des Wirbelstabs (2) hat,
- der Anschlussverbinder (4) ein relativ zu dem Körper (6) des Anschlussverbinders (4) verschiebbares Verschlussorgan (12) aufweist, wobei das Verschlussorgan (12) eine Freigabeposition hat, in der das Verschlussorgan (12) den Durchgang des Wirbelstabs (2) durch die seitliche Öffnung (10) des Lagers zum Empfangen (9) ermöglicht,
- Mittel zur Druckausübung (5) es erlauben, einer Bewegung des Verschlussorgans (12) hin zu seiner Freigabeposition selektiv entgegenzuwirken,
**dadurch gekennzeichnet, dass**:
- das Verschlussorgan (12) relativ zum Körper (6) des Anschlussverbinders (4) von seiner Freigabeposition hin zu einer Rückhalteposition verschiebbar ist, in der das Verschlussorgan (12) dem Durchgang des Wirbelstabs (2) durch die seitliche Öffnung (10) des Lagers zum Empfangen (9) durch elastische Mittel zum Rückstellen (14) elastisch entgegenwirkt, die das Verschlussorgan (12) anliegend gegen den in dem Lager zum Empfangen (9) enthaltenen Wirbelstab (2) gemäß einer ersten vorbestimmten Spannkraft drücken,
- Mittel zur Druckausübung (5) es erlauben, das Verschlussorgan (12) selektiv hin zu einer Verschlussposition zu drängen, in der das Verschlussorgan (12) anliegend gegen den im Lager zum Empfangen (9) enthaltenen Wirbelstab (2) gemäß einer zweiten Spannkraft gedrückt ist, die größer ist als die erste vorbestimmte Spannkraft, die von den elastischen Mitteln zum Rückstellen (14) ausgeübt wird.

2. System für Osteosynthese der Wirbelsäule (1) gemäß Anspruch 1, **dadurch gekennzeichnet, dass**:
- das Verschlussorgan (12) eine Backe (13) umfasst,
- in der Rückhalteposition und in der Verschlussposition die Backe (13) und eine gegenüberliegende Kante (10a) des Lagers zum Empfangen (9) voneinander mit einem Abstand (DR) beabstandet sind, der kleiner als der Durchmesser (D2) des Wirbelstabs (2) ist,
- in der Freigabeposition die Backe (13) und die gegenüberliegende Kante (10a) des Lagers zum Empfangen (9) voneinander mit einem Abstand (DL) beabstandet sind, der größer als der Durchmesser (D2) des Wirbelstabs (2) ist.

3. System für Osteosynthese der Wirbelsäule (1) gemäß einem der Ansprüche 1 oder 2, **dadurch gekennzeichnet, dass** das Verschlussorgan (12) schwenkbar um eine Schwenkachse (17) angeordnet ist, die gemäß einer dritten axialen Richtung (III-III) parallel zur zweiten axialen Richtung (II-II) orientiert ist.

4. System für Osteosynthese der Wirbelsäule (1) gemäß Anspruch 3, **dadurch gekennzeichnet, dass** das Verschlussorgan (12) einen ersten Arm (18) aufweist, der sich radial von der Schwenkachse (17) weg erstreckt und, vorzugsweise benachbart ihres freien Endes (18a), mit einer Immobilisierungsfläche (15) versehen ist gegen welche die Mittel zur Druckausübung (5) anliegend zum Einsatz kommen, um das Verschlussorgan (12) hin zu seiner Verschlussposition zu drängen.

5. System für Osteosynthese der Wirbelsäule (1) gemäß Anspruch 4, **dadurch gekennzeichnet, dass**:
- das Verschlussorgan (12) einen zweiten Arm (19) aufweist, der sich radial von der Schwenkachse (17) von einem festen Ende (19b) weg erstreckt und in einem freien Ende (19a) endet,
- zwischen seinem festen Ende (19b) und seinem freien Ende (19a) der zweite Arm (19) mit einem Abschnitt (19c) besetzt ist, der leichter elastisch verformbar ist als der erste Arm (18) und der nicht in Kontakt mit dem Körper (6) des Anschlussverbinders (4) ist,
- der zweite Arm (19) benachbart seines freien Endes (19a) anliegend am Körper (6) des Anschlussverbinders (4) gelagert und so in Übereinstimmung gebracht ist, dass er das Verschlussorgan (12) elastisch hin zu seiner Rückhalteposition zurückstellt.

6. System für Osteosynthese der Wirbelsäule (1) gemäß einem der Ansprüche 1 bis 5, **gekennzeichnet dadurch, dass** das Lager zum Empfangen (9) intern mit Rückhaltereliefs (20) besetzt ist.

7. System für Osteosynthese der Wirbelsäule (1) gemäß einem der Ansprüche 1 bis 6, **gekennzeichnet dadurch, dass** die Mittel zum Empfangen (7) des Wirbelverankerungsorgans (3) ein Loch (8) umfassen, das den Körper (6) durchsetzt und gemäß der ersten axialen Richtung (I-I) orientiert ist.

8. System für Osteosynthese der Wirbelsäule (1) gemäß einem der Ansprüche 1 bis 7, **dadurch gekennzeichnet, dass**:
- das Wirbelverankerungsorgan (3) einen ersten mit einem Außengewinde versehenen Abschnitt (3a) umfasst, der vorgesehen ist, in einen Wirbel einzudringen, und der in einem Flansch (3b) endet, von dem sich ein zweiter Abschnitt (3c) erstreckt, der vorgesehen ist, die Mittel zur Druckausübung (5) aufzunehmen,
- der Flansch (3b) vorgesehen ist, den Anschlussverbinder (4) durch Drücken mit den Mitteln zur Druckausübung (5) anliegend aufzunehmen.

9. System für Osteosynthese der Wirbelsäule (1) gemäß Anspruch 8, **dadurch gekennzeichnet, dass**:
- der zweite Abschnitt (3c) des Wirbelverankerungsorgans (3) mit einem Außengewinde versehen ist,
- die Mittel zur Druckausübung (5) eine Mutter (21) umfassen, die zum Anschrauben auf den zweiten Abschnitt (3c) des Wirbelverankerungsorgans (3) vorgesehen ist.

## Claims

1. Spinal osteosynthesis system (1) comprising a vertebral rod (2), a vertebral anchoring member (3), a connector (4) between the vertebral rod (2) and the vertebral anchoring member (3), and pressure means (5) for selectively blocking together the vertebral rod (2), the vertebral anchoring member (3) and the connector (4), in which:
- the connector (4) comprises a body (6) having receiving means (7) for receiving the vertebral anchoring member (3) oriented in a first axial direction (I-I),
- the body (6) comprises a substantially cylindrical receiving seat (9) intended to receive the vertebral rod (2) and extending in a second axial direction (II-II) substantially perpendicular to the first axial direction (I-I),
- the receiving seat (9) has a lateral opening (10) defining two edges (10a, 10b) and allowing the vertebral rod (2) to be introduced into the receiving seat (9) transversely or radially with respect to the second axial direction (II-II), the lateral opening (10) having a width (L10) at least equal to the diameter (D2) of the vertebral rod (2),
- the connector (4) has a locking member (12), which is movable with respect to the body (6) of the connector (4), the locking member (12) having a release position, in which the locking member (12) allows the vertebral rod (2) to pass through the lateral opening (10) of the receiving seat (9),
- the pressure means (5) make it possible to selectively oppose a movement of the locking member (12) to its release position,
**characterized in that**:
- the locking member (12) is movable with respect to the body (6) of the connector (4) from its release position to a retention position, in which the locking member (12) elastically opposes the passage of the vertebral rod (2) through the lateral opening (10) of the receiving seat (9) by elastic return means (14) which press the locking member (12) such that it bears against the vertebral rod (2) contained in the receiving seat (9), with a first predetermined clamping force,
- the pressure means (5) make it possible to selectively bias the locking member (12) to a locking position, in which the locking member (12) is pressed so as to bear against the vertebral rod (2) contained in the receiving seat (9), with a second clamping force, which is greater than the first predetermined clamping force exerted by the elastic return means (14).

2. Spinal osteosynthesis system (1) according to Claim 1, **characterized in that**:
- the locking member (12) comprises a jaw (13),
- in the retention position and in the locking position, the jaw (13) and an opposite edge (10a) of the receiving seat (9) are spaced apart from each other by a distance (DR) smaller than the diameter (D2) of the vertebral rod (2),
- in the release position, the jaw (13) and said opposite edge (10a) of the receiving seat (9) are spaced apart from each other by a distance (DL) greater than the diameter (D2) of the vertebral rod (2).

3. Spinal osteosynthesis system (1) according to either of Claims 1 and 2, **characterized in that** the locking member (12) is arranged pivotably about a pivot pin (17), which is oriented in a third axial direction (III-III) parallel to the second axial direction (II-II).

4. Spinal osteosynthesis system (1) according to Claim 3, **characterized in that** the locking member (12) has a first arm (18) extending radially away from the pivot pin (17) and equipped with an immobilizing facet (15), preferably near its free end (18a), against which the pressure means (5) will bear in order to bias the locking member (12) to its locking position.

5. Spinal osteosynthesis system (1) according to Claim 4, **characterized in that**:
- the locking member (12) has a second arm (19) extending radially away from the pivot pin (17) from a fixed end (19b) and terminating with a free end (19a),
- between its fixed end (19b) and its free end (19a), the second arm (19) is provided with a segment (19c) which is more easily elastically deformable than the first arm (18) and which is not in contact with the body (6) of the connector (4),
- the second arm (19) bears, near its free end (19a), on the body (6) of the connector (4) and is configured to return the locking member (12) elastically to its retention position.

6. Spinal osteosynthesis system (1) according to any one of Claims 1 to 5, **characterized in that** the receiving seat (9) is provided internally with retention reliefs (20).

7. Spinal osteosynthesis system (1) according to any one of Claims 1 to 6, **characterized in that** the receiving means (7) for the vertebral anchoring member (3) comprise a hole (8) extending through the body (6) and oriented in the first axial direction (I-I).

8. Spinal osteosynthesis system (1) according to any one of Claims 1 to 7, **characterized in that**:
- the vertebral anchoring member (3) comprises a first externally threaded segment (3a) intended to penetrate a vertebra and terminating with a shoulder (3b), from which there extends a second segment (3c) intended to receive the pressure means (5),
- the shoulder (3b) is intended to receive the connector (4) bearing on it by being pressed by the pressure means (5).

9. Spinal osteosynthesis system (1) according to Claim 8, **characterized in that**:
- the second segment (3c) of the vertebral anchoring member (3) is threaded externally,
- the pressure means (5) comprise a nut (21) intended to be screwed onto the second segment (3c) of the vertebral anchoring member (3).
